# EUROPEAN PATENT APPLICATION

(11) **EP 2 708 181 A1**
(43) Date of publication of application: **19.03.2014**
(21) Application number: 13183167.9
(22) Date of filing: 05.09.2013
(51) Int. Cl.: A61B 5/04, A61B 18/14, A61B 5/0488

(54) **Renal denervation catheter**

(30) Priority: 13.09.2012 JP 2012201653
(71) Applicant: Nihon Kohden Corporation, Shinjuku-ku Tokyo (JP)
(72) Inventor: Nishii, Naoto, Shinjuku-ku, Tokyo (JP)
(74) Representative: Grünecker, Kinkeldey, Stockmair & Schwanhäusser

(57) **Abstract**

A catheter includes: a shaft at least a part of which is to be inserted into a blood vessel; an inlet; an outlet formed in the shaft; a lumen which extends inside the shaft, and through which the inlet and outlet communicate with each other; a balloon disposed on an outer circumference of the shaft, and expandable and contractable; an electrode disposed on a surface of the balloon; a signal acquirer electrically connected to the electrode; a first hole formed in the shaft in a zone between the balloon and the inlet; and a second hole formed in the shaft in a zone between the balloon and the outlet. A blood flowing through the blood vessel is made possible to flow in one of the first and second holes, and flow out from the other hole.

## Description

### CROSS-REFERENCE TO RELATED APPLICATION(S)

This application is based upon and claims the benefit of priority from prior Japanese patent application No. 2012-201653, filed on September 13, 2012, the entire contents of which are incorporated herein by reference.

### BACKGROUND

The presently disclosed subject matter relates to a catheter a part of which is to be inserted into a blood vessel of a living body.

As a catheter of this kind, there is that which is used for renal artery ablation as treatment for refractory hypertension (for example, see JP-A-2012-110748). Renal artery ablation is a technique in which a high-frequency voltage pulse is applied through electrodes disposed in an ablation catheter that is inserted into a renal artery, and the renal sympathetic nerves surrounding the adventitia of the renal artery are cauterized. The ablation is performed based on parameters (the value of the voltage, the duration of the voltage application, and the like) which are experimentally derived.

When renal artery ablation is performed, the blood pressure decreases not immediately after the surgery procedure but after several months. As described above, the ablation is performed based on values which are experimentally derived. Therefore, the success or failure of nerve cauterization cannot be evaluated during or immediately after the surgery procedure. In the case where the pressure decrease effect does not appear after several months, it is difficult to determine whether the phenomenon is caused by insufficient nerve cauterization or by another disease of the patient.

### SUMMARY

The presently disclosed subject matter may provide a technique for enabling the success or failure of nerve cauterization to be determined during a procedure of renal artery ablation, and more specifically, provide a technique in which the status of a target of surgery using a catheter is made able to be monitored, thereby enabling the success or failure of the surgery to be determined during the surgery procedure.

The catheter may comprise: a shaft at least a part of which is to be inserted into a blood vessel of a living body; a first inlet; a first outlet which is formed in the shaft; a first lumen which extends inside the shaft, and through which the first inlet and the first outlet communicate with each other; a balloon which is disposed on an outer circumference of the shaft, and which is expandable and contractable; an electrode which is disposed on a surface of the balloon; a signal acquirer which is electrically connected to the electrode; a first hole which is formed in the shaft in a zone between the balloon and the first inlet; and a second hole which is formed in the shaft in a zone between the balloon and the first outlet, wherein a blood flowing through the blood vessel is made possible to flow in one of the first and second holes, and flow out from the other of the first and second holes.

The first and second holes may communicate with the first lumen.

The catheter may further comprise: a second inlet; a second outlet which is formed in the shaft; and a second lumen which extends inside the shaft, and through which the second inlet and the second outlet communicates with each other. The second outlet may be opened in the zone between the balloon and the first outlet.

An air passage which communicates with an interior of the balloon may be formed in the shaft.

The catheter may further comprise: an ablation catheter which is insertable from the first inlet to the first outlet through the first lumen.

An electrode which is energizable to perform ablation may be disposed on the surface of the balloon.

### BRIEF DESCRIPTION OF THE DRAWINGS

Fig. 1 is a view diagrammatically showing the appearance of a catheter of an embodiment of the presently disclosed subject matter.
Figs. 2A and 2B are sectional views diagrammatically showing the internal structure of a shaft, respectively taken along lines IIA-IIA and IIB-IIB in Fig. 1.
Fig. 3 is a view diagrammatically showing the internal structure of the shaft which is inserted into a blood vessel.
Figs. 4A and 4B are views diagrammatically showing modifications of the catheter.

### DETAILED DESCRIPTION OF EXEMPLARY EMBODIMENTS

An embodiment of the presently disclosed subject matter will be described in detail with reference to the accompanying drawings. In the drawings, the scale is adequately changed in order to draw components in a recognizable size.

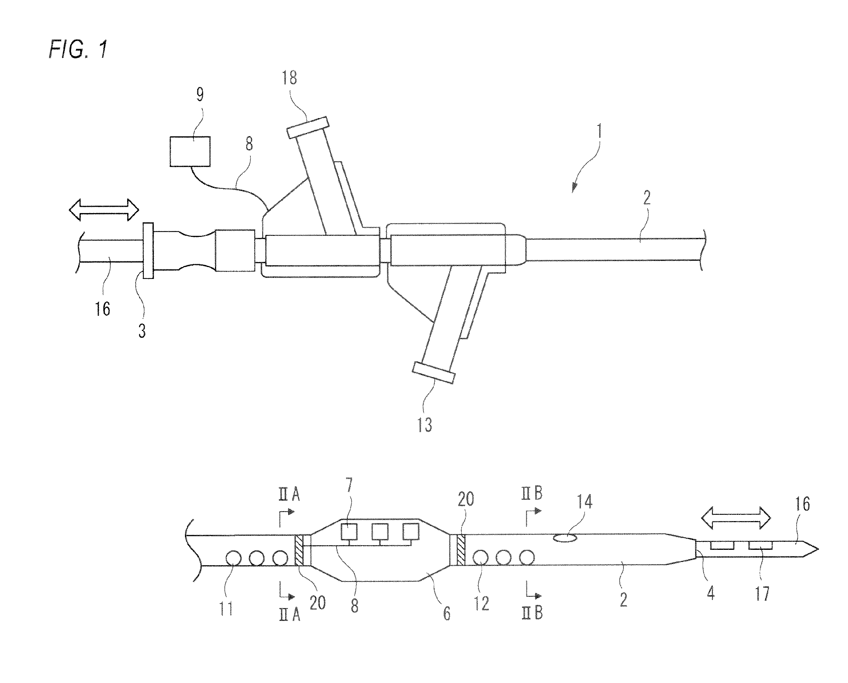

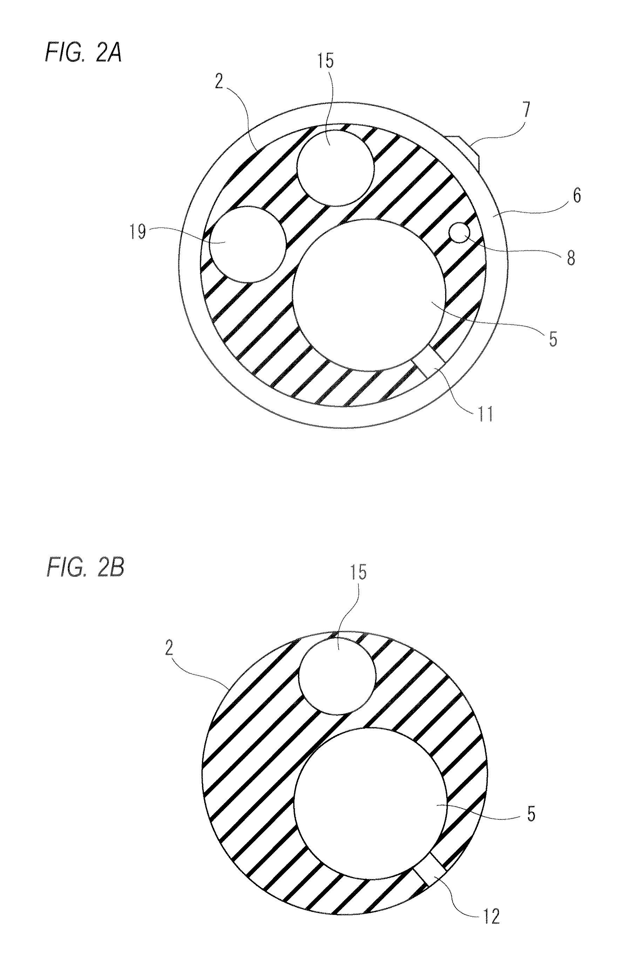

Fig. 1 diagrammatically shows the appearance of a catheter 1 of an embodiment of the presently disclosed subject matter. Figs. 2A and 2B are sectional views diagrammatically showing the internal structure of the catheter 1, respectively taken along lines IIA-IIA and IIB-IIB in Fig. 1.

The catheter 1 includes a shaft 2 at least a part of which is to be inserted into a blood vessel of a living body. The shaft 2 is long and thin, and therefore a part of the shaft is not shown in the figure. The shaft 2 has flexibility to be bendable.

A first inlet 3 is opened on the side of the basal end of the shaft 2. A first outlet 4 is opened in the tip end of the shaft 2. A first lumen 5 through which the first inlet 3 and the first outlet 4 communicate with each other extends inside the shaft 2.

A balloon 6 which is made of a flexible material is disposed on the outer circumference of the tip end side of the shaft 2. The balloon 6 is made of a flexible material to be expandable and contractable.

A plurality of electrodes 7 are disposed on the surface of the balloon 6. A signal line 8 which is connected to the electrodes 7 extends from the surface of the balloon 6 to the outer circumferential surface of the shaft 2, and enters the interior of the shaft 2. The signal line 8 which extends inside the shaft 2 as shown in Figs. 2A and 2B is led out to the outside in the base end side of the shaft 2 as shown in Fig. 1, and connected to a connector 9. Namely, the connector 9 is electrically connected to the electrodes 7 through the signal line 8.

In a zone between the balloon 6 and the first inlet 3, a plurality of first holes 11 are formed in the shaft 2. In a zone between the balloon 6 and the first outlet 4, a plurality of second holes 12 are formed in the shaft 2. The first holes 11 and the second holes 12 communicate with the first lumen 5.

A second inlet 13 is opened on the side of the basal end of the shaft 2. A second outlet 14 is formed in the shaft 2, and opened in the zone between the balloon 6 and the first outlet 4. A second lumen 15 through which the second inlet 13 and the second outlet 14 communicate with each other extends inside the shaft 2.

An ablation catheter 16 is insertable from the first inlet 3 to the first outlet 4 through the first lumen 5. A plurality of electrodes 17 are disposed in a tip end portion of the ablation catheter 16. The ablation catheter 16 in the embodiment is used for applying a high-frequency voltage pulse to the renal parasympathetic nerves through the electrodes 17, to cauterize the nerves.

When renal artery ablation is to be performed, a small-diameter guide wire which is not shown is inserted from the first inlet 3 to the first outlet 4 through the first lumen 5. The guide wire is inserted into the renal artery, and the balloon 6 is guided to the vicinity of the renal parasympathetic nerve on which the surgery is to be performed. As shown in Fig. 1, a marker 20 which blocks X-rays is formed in the vicinity of the balloon 6, and therefore the correct position of the balloon 6 in the blood vessel can be acquired via an x-ray image.

Then, the shaft 2 is inserted into the renal artery, and advanced along the guide wire, thereby causing the tip end of the shaft 2 to be placed in the vicinity of the renal parasympathetic nerve on which the surgery is to be performed. Here, the guide wire is pulled out through the first inlet 3, and instead the ablation catheter 16 is inserted into the first inlet.

The ablation catheter 16 advances in the first lumen 5, and projects from the first outlet 4. In the ablation catheter 16, the positions of the electrodes 17 with respect to the site to be subject to surgery can be adjusted by pushing and pulling the ablation catheter 16 on the side of the first inlet 3.

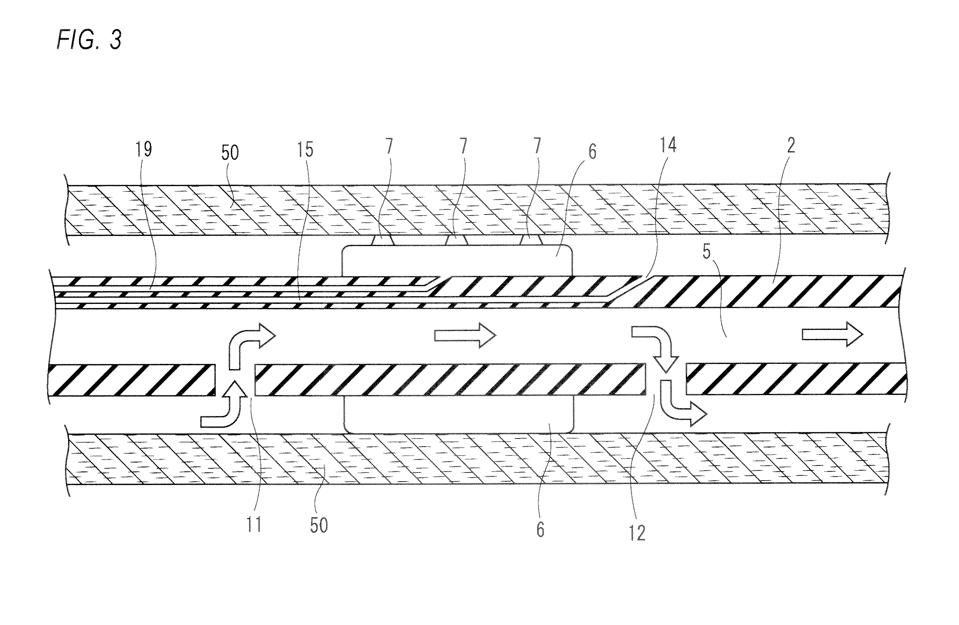

A pressure port 18 is disposed on the side of the basal end of the shaft 2. The pressure port 18 communicates with an air passage 19 which is formed in the shaft 2. As shown in Fig. 3, the air passage 19 communicates with the interior of the balloon 6. A syringe which is not shown is attached to the pressure port 18. When a pressurizing operation is performed on the syringe, the balloon 6 is expanded, and, when a depressurizing operation is performed, the balloon 6 is contracted.

Fig. 3 is a view showing the positional relationship of the renal artery wall 50 and the catheter 1 in the case where renal artery ablation is performed, together with the internal structure of the catheter 1. In the figure, the ablation catheter 16 is not illustrated, and one first hole 11 and one second hole 12 are shown.

When the electrodes 17 of the ablation catheter 16 are placed in positions where the renal sympathetic nerves which are to be subject to the surgery can be cauterized, a pressurizing operation is performed on the syringe attached to the pressure port 18, and the balloon 6 is expanded. At this time, the electrodes 7 disposed on the surface of the balloon 6 are in close contact with the inner wall of the renal artery wall 50. According to the configuration, irrespective of the size of a blood vessel which depends on a living body, the potential of the renal sympathetic nerves can be detected in the form of the potential of the electrodes 7. A signal indicating of the potential of the renal sympathetic nerves is acquired in the connector 9 which is an example of a signal acquirer, through the signal line 8.

When the connector 9 is connected to an adequate measuring apparatus, the surgery can be performed while monitoring the status of the renal sympathetic nerves. If it is determined, from the state of the potential signal, that the nerve cauterization is insufficient, the application duration and voltage value of the high-frequency voltage pulse which is applied through the electrodes 17 of the ablation catheter 16 are adequately adjusted. Even when a follow-up for several months is not performed after the surgery procedure, therefore, the success or failure of nerve cauterization can be evaluated during the surgery of renal artery ablation.

As shown in Fig. 3, the blood flowing through the blood vessel can flow in from the first hole 11, pass through the first lumen 5, and then flow out from the second hole 12. Therefore, a flow path for blood which connects the upstream and downstream sides of the balloon 6 with each other can be ensured in the shaft 2. In order to correctly acquire the potential of the renal sympathetic nerves, it is necessary to sufficiently expand the balloon 6 to cause the electrodes 7 to be in close contact with the inner wall of the renal artery. According to the configuration of the embodiment, it is possible to avoid the situation where the blood flow directed to the kidney is blocked by the thus expanded balloon 6 and the kidney malfunctions.

Therefore, renal artery ablation can be surely performed while ensuring a blood supply to the kidney and monitoring the status of the renal sympathetic nerves.

In the embodiment, the first and second holes 11, 12 communicate with the first lumen 5, and hence a part of the first lumen 5 is used as a detour for a blood flow. Although the detour is disposed, therefore, it is possible to prevent the shaft 2 from being enlarged.

For example, a syringe which is not shown is attached to the second inlet 13, and a medical solution is poured into the syringe. The medical solution is discharged from the second outlet 14 through the second lumen 15, and sent to the kidney. Since the second outlet 14 is opened in the downstream side of the balloon 6, the supply of the medical solution is not blocked by the expanded balloon 6. Even during a surgery procedure for renal artery ablation involving monitoring of the renal parasympathetic nerves, therefore, a required supply of the medical solution can be continued.

In the embodiment, the air passage 19 which communicates with the interior of the balloon 6 is formed inside the shaft 2. When the shaft 2 inserted into the blood vessel is moved, therefore, the air passage can be prevented from being damaged, and the balloon 6 can be surely expanded during a surgery procedure. Consequently, monitoring of the renal sympathetic nerves through the electrodes 7 can be surely performed.

The embodiment has been described in order to facilitate understanding of the presently disclosed subject matter, and is not intended to limit the presently disclosed subj ect matter. It is a matter of course that the presently disclosed subject matter may be changed or improved without departing the spirit thereof, and includes equivalent embodiments.

The number, position, and shape of the first holes 11 are not limited to those shown in Fig. 1. They may be adequately determined in accordance with the specifications as far as at least one first hole is disposed in a zone between the first inlet 3 and the balloon 6.

The number, position, and shape of the second holes 12 are not limited to those shown in Fig. 1. They may be adequately determined in accordance with the specifications as far as at least one second hole is disposed in the zone between the balloon 6 and the first outlet 4.

The first holes 11 and the second holes 12 are not always required to communicate with the first lumen 5. As far as a blood flow path which detours in the shaft 2 can be ensured, the holes may be configured so as to communicate with the second lumen 15. Alternatively, a configuration may be employed where another lumen which is different from the first and second lumen 5, 15 is formed in the shaft 2, and the first and second holes 11, 12 communicate with the other lumen.

The blood is not always required to flow in from the first holes 11 and flow out from the second holes 12. As far as a blood flow path which detours in the shaft 2 can be ensured, a configuration may be employed where, in accordance with the contents of the surgery procedure or the blood vessel into which the shaft 2 is to be inserted, the blood flows in from the second holes 12 and flows out from the first holes 11.

The first lumen 5 is not always required to be used in the insertion of the ablation catheter 16. For example, the first lumen 5 may be filled with physiological saline, and the catheter 1 may be used for measuring the blood pressure.

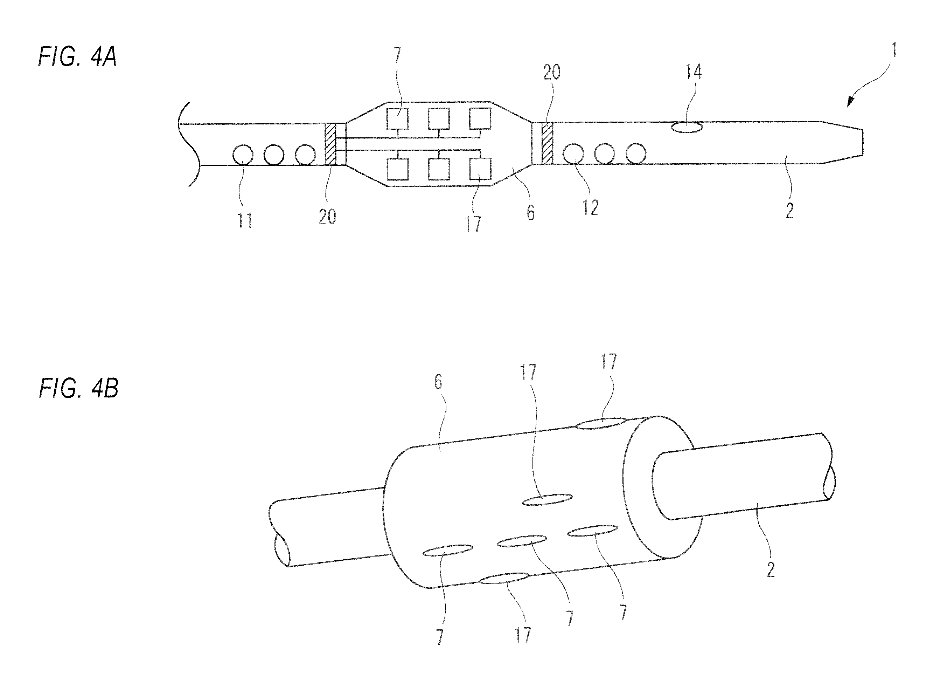

In addition to or in place of the insertion of the ablation catheter 16 into the first lumen 5, a configuration may be employed where the electrodes 17 which is energizable for ablation are disposed on the surface of the balloon 6 as shown in Fig. 4A.

In the electrodes 7, 17, the numbers and the places on the balloon 6 may be arbitrarily determined. When the electrodes 17 are disposed in a plurality of places in the circumferential direction of the balloon 6 as shown in Fig. 4B, ablation of the target can be performed more surely.

The electrodes 7 are not always required to be used for acquiring the potential of the renal sympathetic nerves. In order to monitor the status of a target of surgery using a catheter, the electrodes may be used for an appropriate purpose.

When the second inlet 13, the second outlet 14, and the second lumen 15 are not particularly necessary, they may be omitted. The air passage 19 may be disposed outside the shaft 2.

According to an aspect of the presently disclosed subject matter, when the balloon is expanded, the electrode disposed on the surface of the balloon is caused to be in close contact with the inner wall of a blood vessel in the vicinity of the surgery target, and the catheter can placed in a desired position. Irrespective of the size of a blood vessel of a living body, therefore, the potential of the surgery target can be detected in the form of the potential of the electrode. When a signal indicative of the potential is acquired by a signal acquirer, the status of the surgery target can be kept monitored also during the surgery procedure, and the success or failure of the surgery procedure can be determined.

The blood flowing through the blood vessel flows in from one of the first and second holes, and flows out from the other hole. Therefore, a flow path for the blood, which connects the upstream and downstream sides of the balloon can be ensured in the shaft. In order to correctly acquire the potential of the surgery target, particularly, it is required that the balloon is sufficiently expanded and the electrodes are in close contact with the inner wall of the blood vessel. According to an aspect of the presently disclosed subject matter, however, it is possible to avoid a situation where the blood flow is blocked by the expanded balloon.

In a case where the first and second holes communicate with the first lumen, a part of the first lumen is used as a detour for a blood flow. While the detour is disposed, therefore, it is possible to prevent the shaft from being enlarged.

In a case where the catheter further include: a second inlet; a second outlet which is formed in the shaft; and a second lumen which extends inside the shaft, and through which the second inlet and the second outlet communicates with each other, and the second outlet is opened in the zone between the balloon and the first outlet, the second lumen can be used for a purpose which is different from that of the first lumen, such as a supply of a medical solution. In the case where the blood flows from the side of the first inlet toward the first outlet, a supply of a medical solution or the like is not blocked by the expanded balloon because the second outlet is opened on the downstream side of the balloon. Even during a surgery procedure involving status monitoring of the surgery target, therefore, a supply of a medical solution or the like can be continued.

In a case where an air passage which communicates with an interior of the balloon is formed in the shaft, when the shaft inserted into the blood vessel is moved, the air passage can be prevented from being damaged, and the balloon can be surely expanded during a surgery procedure. Therefore, monitoring of the surgery target through the electrode can be surely performed.

In a case where the catheter further includes an ablation catheter which is insertable from the first inlet to the first outlet through the first lumen, the ablation catheter can be used for cauterization of the renal parasympathetic nerves. At this time, while ensuring a blood supply to the kidney and monitoring the status of the renal sympathetic nerves, renal artery ablation can be surely performed.

A configuration may be employed where an electrode which is energizable to perform ablation is disposed on the surface of the balloon in addition to or in place of the ablation catheter.

## Claims

1. A catheter comprising:
a shaft at least a part of which is to be inserted into a blood vessel of a living body;
a first inlet;
a first outlet which is formed in the shaft;
a first lumen which extends inside the shaft, and through which the first inlet and the first outlet communicate with each other;
a balloon which is disposed on an outer circumference of the shaft, and which is expandable and contractable;
an electrode which is disposed on a surface of the balloon;
a signal acquirer which is electrically connected to the electrode;
a first hole which is formed in the shaft in a zone between the balloon and the first inlet; and
a second hole which is formed in the shaft in a zone between the balloon and the first outlet, wherein
a blood flowing through the blood vessel is made possible to flow in one of the first and second holes, and flow out from the other of the first and second holes.

2. The catheter according to claim 1, wherein the first and second holes communicate with the first lumen.

3. The catheter according to claim 1 or 2, further comprising:
a second inlet;
a second outlet which is formed in the shaft; and
a second lumen which extends inside the shaft, and through which the second inlet and the second outlet communicates with each other, wherein
the second outlet is opened in the zone between the balloon and the first outlet.

4. The catheter according to any one of claims 1 through 3, wherein an air passage which communicates with an interior of the balloon is formed in the shaft.

5. The catheter according to any one of claims 1 through 4, further comprising: an ablation catheter which is insertable from the first inlet to the first outlet through the first lumen.

6. The catheter according to any one of claims 1 through 5, wherein an electrode which is energizable to perform ablation is disposed on the surface of the balloon.
